# EUROPÄISCHE PATENTANMELDUNG

(11) **EP 0 814 083 A2**
(43) Veröffentlichungstag der Anmeldung: **29.12.1997**
(21) Anmeldenummer: 97108098.1
(22) Anmeldetag: 20.05.1997
(51) Int. Cl.: C07D 311/16, C07C 69/716

(54) **Verfahren zur Herstellung von 3-(7- Diethylamino-2-oxo-2H-chromen-3-yl)-3-oxopropionsäure-(1-methoxy-2-propylester)**

(30) Priorität: 23.05.1996 DE 19620747
(71) Anmelder: Clariant GmbH, 65929 Frankfurt am Main (DE)
(72) Erfinder: Koch, Peter, Dr., 63179 Obertshausen (DE); Bauer, Wolfgang, Dr., 63477 Maintal (DE)

(57) **Zusammenfassung**

Die vorliegende Erfindung betrifft Verfahren zur Herstellung von 3-(7-Diethylamino-2-oxo-2H-chromen-3-yl)-3-oxopropionsäure-(1-methoxy-2-propylester) der Formel I 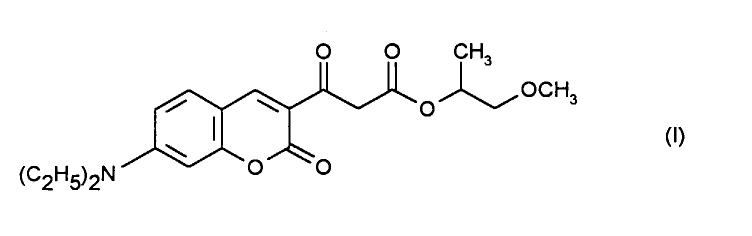 sowie Acetondicarbonsäuredi-(1-methoxy-2-propylester) als Zwischenprodukt für eines der Verfahren.

## Beschreibung

Die vorliegende Erfindung betrifft Verfahren zur Herstellung von 3-(7-Diethylamino-2-oxo-2H-chromen-3-yl)-3-oxopropionsäure-(1-methoxy-2-propylester) der Formel I 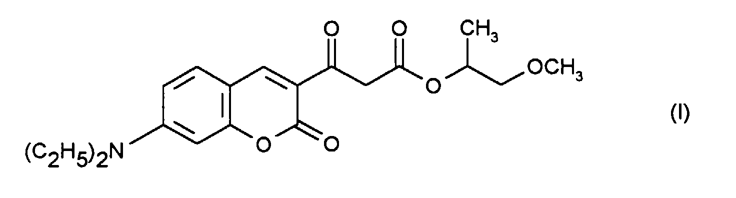 sowie Acetondicarbonsäuredi-(1-methoxy-2-propylester) als neues Zwischenprodukt für eines der Verfahren.

Der Methoxypropylester der Formel I findet Verwendung als Farbstoff für Photoresists, insbesondere Photoresists auf Basis von Novolak-Harzen und Chinondiaziden. Gegenüber dem entsprechenden Methylester, der ebenfalls als Farbstoff für Photoresists Verwendung findet, zeichnet sich der Methoxypropylester der Formel I durch günstigere Löslichkeitseigenschaften aus. Nähere Angaben zum Ester der Formel I und seiner Verwendung finden sich in der Patentschrift US-A-5 225 312, deren Inhalt hierzu in vollem Umfang Bestandteil der vorliegenden Offenbarung ist.

In der US-A-5 225 312 wird ein Verfahren zur Herstellung von I beschrieben, bei dem der entsprechende Methylester der Formel II, der gemäß der DE-C-2 704 368 aus 4-Diethylaminosalicylaldehyd und Acetondicarbonsäuredimethylester unter Basenkatalyse erhältlich ist, mit 1-Methoxy-2-propanol der Formel III in Gegenwart von Schwefelsäure als Katalysator unter Abdestillieren von Methanol umgeestert wird.

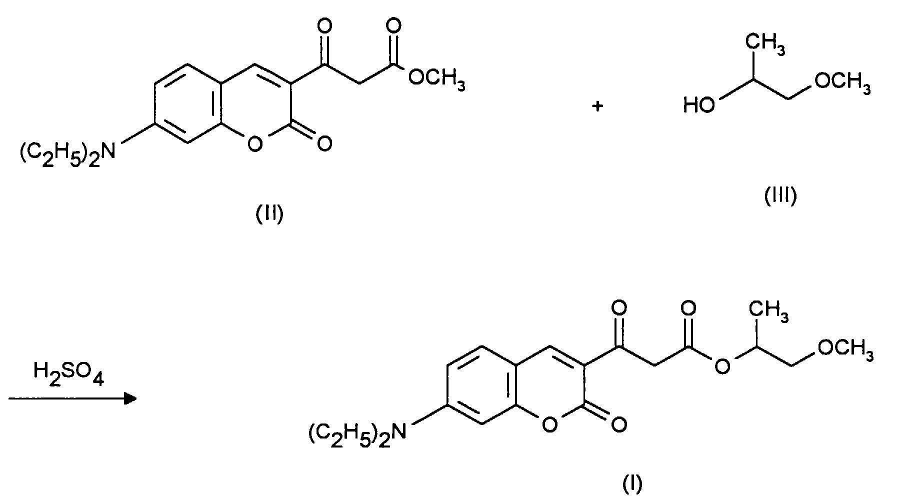

Das Produkt dieser Umesterung, das nach Neutralisation der Schwefelsäure mit Morpholin durch Fällen mit Wasser, Filtration und Waschen isoliert wird, besteht zum überwiegenden Teil aber noch aus nicht umgeestertem Methylester der Formel II. Gemäß der US-A-5 225 312 ist ein derartiges Produkt aber für die Verwendung in Photoresists geeignet und wird hierfür eingesetzt. Es weist wegen seiner besseren Löslichkeit z.B. in Ethyllactat Vorteile gegenüber dem reinen Methylester der Formel II auf.

Das aus der US-A-5 225 312 bekannte Verfahren zur Herstellung von I bzw. zur Herstellung eines Gemisches aus I und II weist allerdings eine Reihe von Nachteilen auf. So werden mit 26.4 Mol III pro Mol II und 1.27 Mol Schwefelsäure pro Mol II große Überschüsse an Umesterungsalkohol und Katalysator benötigt. Aufgrund der großen Menge 1-Methoxy-2-propanol und der guten Löslichkeit des erhaltenen Produkts in diesem Lösungsmittel wird zum Fällen ein großes Wasservolumen benötigt, was eine schlechte Raumausbeute von ca. 26 l pro kg Umesterungsprodukt zur Folge hat. Durch die wäßrige Aufarbeitung fällt ein Abwasser an, das 1-Methoxy-2-propanol, Morpholin und weitere organische Bestandteile enthält und aufgearbeitet oder entsorgt werden muß.

Die Ausbeute an Umesterungsprodukt beträgt gemäß der US-A-5 225 312 nur 86.7 Gewichtsprozent, bezogen auf das Gewicht des eingesetzten Methylesters der Formel II, dabei enthält das Produkt neben den Estern I und II aber noch zahlreiche Verunreinigungen, wie sich bei seiner Untersuchung durch Hochdruckflüssigkeitschromatographie (HPLC) zeigt. Zudem ist der erzielbare Umesterungsgrad sehr niedrig. Die HPLC ergibt, daß das Produkt auf 1 Teil Verbindung I ungefähr 2.7 Teile Verbindung II enthält, nicht umgeestertes Ausgangsmaterial überwiegt also bei weitern.

Aufgabe der vorliegenden Erfindung ist daher die Bereitstellung neuer Verfahren zur Herstellung des Methoxypropylesters der Formel I, die die Nachteile des bekannten Verfahrens vermeiden.

Überraschend wurde gefunden, daß diese Aufgabe gelöst wird, wenn die in der US-A-5 225 312 beschriebene Umesterung des Methylesters der Formel II mit dem Alkohol III ohne Zusatz eines Katalysators durchgeführt wird. Umesterungsreaktionen benötigen generell eine Säuren- oder Basenkatalyse, siehe z.B. J. March, Advanced Organic Chemistry, 3. Aufl., Seite 351, Wiley, New York 1985. Umesterungen unter neutralen Bedingungen sind nur selten beschrieben und benötigen meistens spezielle Hilfsstoffe, z.B. Phosphorylide (siehe Tetrahedron Lett. 21 (1980), 2857), das Addukt aus Azodicarbonsäurediethylester und Triphenylphosphin (siehe Tetrahedron Lett. 1975, 3871) oder Iodtrimethylsilan (siehe Synthesis 1981, 142). Bestimmte spezielle Ester können unter neutralen Bedingungen umgeestert werden, ohne daß ein Hilfsstoff zugesetzt wird, die Reaktionsgeschwindigkeiten sind aber meist klein. So können z.B. enolisierbare Ester wie Acetessigester unter neutralen Bedingungen umgeestert werden, wobei die Bedingungen bei diesen Substraten aber aufgrund der aciden Wasserstoffatome in enolisierbaren Verbindungen als nicht neutral, sondern als sauer angesehen werden können; der Mechanismus, nach dem solche Umesterungen ablaufen, ist aber nicht gesichert (siehe J. Am. Chem. Soc. 73 (1951), 4195 und 74 (1952), 3992).

α,α-Disubstituierte Acetessigester, die nicht enolisierbar sind, lassen sich auf diese Weise nicht umestern (J. Chem. Soc. 89 (1906), 381). Die unter neutralen Bedingungen erfolgende Umesterung von Estern starker organischer Säuren, z.B von Oxalsäureestern oder Fumarsäureestern, wird durch induktive Effekte erklärt (siehe J. Am. Chem. Soc. 74 (1952), 3992).

Daß sich der Propionsäureester der Formel II unter neutralen Bedingungen mit 1-Methoxy-2-propanol umestern läßt, war aufgrund dieses Standes der Technik nicht zu erwarten. Vergleichbare induktive Effekte wie bei Oxalsäureestern oder Fumarsäureestern liegen bei II nicht vor. Eine durch die Wasserstoffatome der enolisierbaren β-Ketoesterfunktion bewirkte Säurekatalyse kann ebenfalls nicht für den beobachteten Effekt verantwortlich sein, da mit der Diethylaminogruppe im Molekül der Formel II ein basisches Zentrum vorhanden ist, durch das die Enol-Protonen neutralisiert werden. Die Diethylaminogruppe kann andererseits aber auch nicht als Base die Umesterung katalysieren, denn für eine effiziente Basenkatalyse sind starke Basen erforderlich, die das 1-Methoxy-2-propanol in sein Anion überführen können. Die Basizität der Aminogruppe reicht hierfür nicht aus. Die beobachtete Umesterung ohne Zusatz eines Katalysators ist also in höchsten Maße überraschend.

Gegenstand der vorliegenden Erfindung ist somit ein Verfahren zur Herstellung von 3-(7-Diethylamino-2-oxo-2H-chromen-3-yl)-3-oxopropionsäure-(1-methoxy-2-propylester) der Formel I oder von Gemischen aus 3-(7-Diethylamino-2-oxo-2H-chromen-3-yl)-3-oxopropionsäure-(1-methoxy-2-propylester) der Formel I und 3-(7-Diethylamino-2-oxo-2H-chromen-3-yl)-3-oxopropionsäuremethylester der Formel II durch Umesterung von 3-(7-Diethylamino-2-oxo-2H-chromen-3-yl)-3-oxopropionsäuremethylester der Formel II mit 1-Methoxy-2-propanol der Formel III, 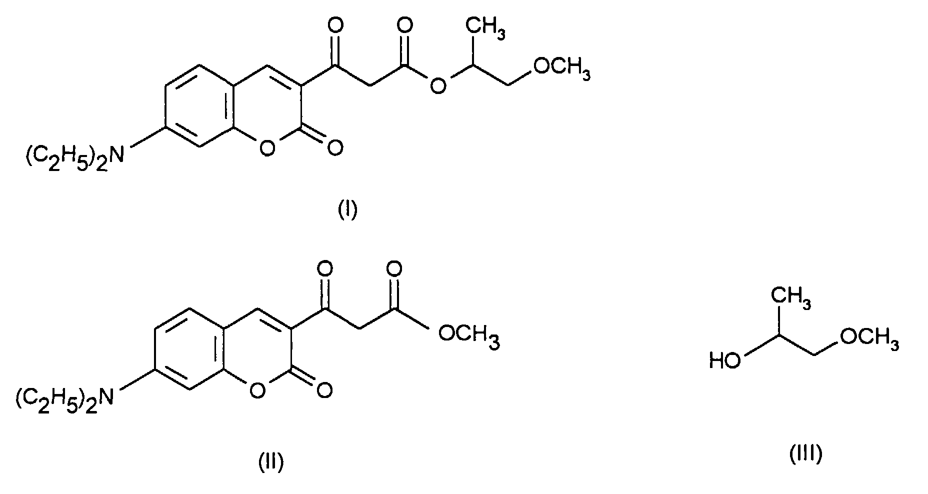 dadurch gekennzeichnet, daß bei der Umesterung kein Katalysator zugesetzt wird.

Die Umesterung wird in der üblichen Weise bei erhöhten Temperaturen durchgeführt. Sie kann in Gegenwart eines zusätzlichen inerten Lösungs- oder Verdünnungsmittels durchgeführt werden, bevorzugt wird sie aber ohne Zusatz eines solchen Lösungs- oder Verdünnungsmittels durchgeführt. Im allgemeinen werden der Methylester der Formel II und das 1-Methoxy-2-propanol der Formel III im gewünschten Mengenverhältnis gemischt, das Gemisch wird unter Rühren auf die gewünschte Reaktionstemperatur erhitzt und bei dieser Temperatur gehalten, bis die Umsetzung beendet oder der gewünschte Umsetzungsgrad erreicht ist. Es kann aber beispielsweise auch das 1-Methoxy-2-propanol bei erhöhter Temperatur vorgelegt werden und in der Hitze der Methylester der Formel II zugesetzt werden. Das bei der Umsetzung entstehende Methanol kann im Reaktionsgemisch verbleiben oder während der Umesterung abdestilliert werden. Bevorzugt wird es nicht abdestilliert. Die Reaktion wird bevorzugt bei 80 bis 150°C, besonders bevorzugt bei 90 bis 130°C, ganz besonders bevorzugt bei 100 bis 120°C durchgeführt. Sie kann bei Atmoshärendruck oder bei Überdruck, insbesondere bei einem Druck zwischen Atmosphärendruck und 6 bar, durchgeführt werden, ebenso kann sie aber auch, insbesondere wenn das entstehende Methanol abdestilliert werden soll, bei Unterdruck, d.h. unter Anlegen eines Vakuums, durchgeführt werden. Bevorzugt wird die Umesterung bei Atmosphärendruck durchgeführt. Für die Umsetzung werden im allgemeinen 1 bis 4 Stunden benötigt. Der Endpunkt der Umsetzung bzw. das Erreichen des gewünschten Umsetzungsgrades kann beispielsweise durch chromatographische Untersuchung des Reaktionsgemisches erfolgen.

Das Mengenverhältnis Methylester der Formel II zu 1-Methoxy-2-propanol der Formel III bei der Reaktion richtet sich unter anderem nach dem gewünschten Umsetzungsgrad. Für die Verwendung in Photoresists wird wie gemäß der US-A-5 225 312 vielfach kein reiner 1-Methoxy-2-propylester der Formel I eingesetzt, sondern ein Gemisch aus diesem und dem Methylester der Formel II. Beim Arbeiten nach dem erfindungsgemäßen Umesterungsverfahren können vergleichbare oder höhere Umesterungsgrade als nach dem Verfahren der US-A-5 225 312 bereits mit wesentlich geringeren Überschüssen an 1-Methoxy-2-propanol erzielt werden. Bevorzugt wird die Umesterung mit 5 bis 10 Mol, besonders bevorzugt mit 6 bis 8 Mol 1-Methoxy-2-propanol der Formel III pro Mol Methylester der Formel II durchgeführt. Das 1-Methoxy-2-propanol kann als Racemat, in Form der reinen Enantiomeren oder als Gemisch der Enantiomeren in beliebigen Mengenverhältnissen eingesetzt werden.

Der Vorteil des erfindungsgemäßen Verfahrens, daß nur ein relativ geringer Überschuß an 1-Methoxy-2-propanol für die Umesterung benötigt wird, ermöglicht auch eine gegenüber dem Stand der Technik verbesserte Aufarbeitung. Die Isolierung des Umesterungsproduktes, also des 1-Methoxy-2-propylesters der Formel I bzw. des Gemisches aus diesem und dem Methylester der Formel II, aus dem Reaktionsgemisch kann wie nach der US-A-5 225 312 durch Fällen mit Wasser und Abfiltrieren des ausgefallenen Produktes erfolgen. Aufgrund des geringeren Überschusses an 1-Methoxy-2-propanol kann dabei auch die Wassermenge verringert werden. Bevorzugt wird das Reaktionsgemisch der Umesterung aber nicht-wäßrig aufgearbeitet. So kann es in einfacher Weise nach Beendigung der Umesterungsreaktion abgekühlt werden, bevorzugt auf eine Temperatur von 0 bis 20°C, besonders bevorzugt auf eine Temperatur von 3 bis 5°C, und dann das auskristallisierte Produkt durch Filtrieren oder Zentrifugieren isoliert werden. Gewünschtenfalls kann vor dem Abkühlen auch ein Teil des 1-Methoxy-2-propanols bzw. des Gemisches aus 1-Methoxy-2-propanol und Methanol abdestilliert werden, z.B. durch teilweises Einengen im Vakuum. Bei einer besonders bevorzugten Aufarbeitungsmethode wird das Reaktionsgemisch - gewünschtenfalls nach teilweisem Einengen - mit einem organischen Lösungsmittel versetzt, in dem das Produkt schwer löslich ist. Der Zusatz des organischen Lösungsmittels kann bereits in der Hitze erfolgen, um die Kristallisation des Produkts zu verbessern. Ganz besonders bevorzugt wird das Reaktionsgemisch der Umesterung mit Methanol versetzt und dann auf 0 bis 20°C, vorteilhaft insbesondere auf 3 bis 5°C, abgekühlt. Das Produkt fällt dabei in einer besonders gut filtrierbaren und waschbaren Form an. Das auskristallisierte Produkt wird dann durch Filtration oder Zentrifugation isoliert, mit Methanol gewaschen und getrocknet. Es resultiert ein gelbes Kristallpulver. Fällt bei der Umesterung ein Gemisch aus dem 1-Methoxy-2-propylester I und dem Methylester II an und soll die Verbindung I in reiner Form hergestellt werden, so kann das Gemisch nach üblichen, dem Fachmann bekannten Methoden, beispielsweise durch Umkristallisieren oder Chromatographie, aufgetrennt werden.

Der nach dem erfindungsgemäßen Verfahren hergestellte 1-Methoxy-2-propylester der Formel I bzw. das Gemisch aus dem 1-Methoxy-2-propylester der Formel I und dem Methylester der Formel II zeichnet sich durch eine hohe Reinheit aus. Im Gegensatz zu dem gemäß der US-A-5 225 312 erhaltenen Produkt sind nach dem Hochdruckflüssigkeitschromatogramm praktisch keine Verunreinigungen vorhanden. Zudem wird nach dem erfindungsgemäßen Verfahren das Umesterungsprodukt in einer hohen Ausbeute von ca. 100 Gewichtsprozent, bezogen auf das Gewicht des eingesetzten Methylesters der Formel II, erhalten, und die Raumausbeute liegt bei unter 5 l/kg. Auf einfache Weise wird nach dem erfindungsgemäßen Verfahren ein Umesterungsgrad von über 50 % erzielt.

Gegenstand der Erfindung ist weiterhin ein Verfahren zur Herstellung von 3-(7-Diethylamino-2-oxo-2H-chromen-3-yl)-3-oxopropionsäure-(1-methoxy-2-propylester) der Formel I oder von Gemischen aus 3-(7-Diethylamino-2-oxo-2H-chromen-3-yl)-3-oxopropionsäure-(1-methoxy-2-propylester) der Formel I und 3-(7-Diethylamino-2-oxo-2H-chromen-3-yl)-3-oxopropionsäurealkylester der allgemeinen Formel IV, in der R für (C₁-C₄)-Alkyl steht, 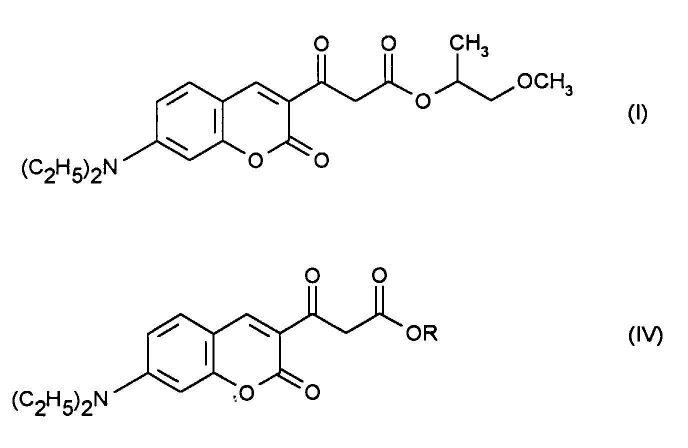 dadurch gekennzeichnet, daß zunächst ein Acetondicarbonsäuredialkylester der allgemeinen Formel V, in der R für (C₁-C₄)-Alkyl steht, mit 1-Methoxy-2-propanol der Formel III zu Acetondicarbonsäuredi-(1-methoxy-2-propylester) der Formel VI oder zu einem Gemisch aus dem Ester der Formel VI, dem Ester der allgemeinen Formel V und dem entsprechenden gemischten Ester umgeestert wird 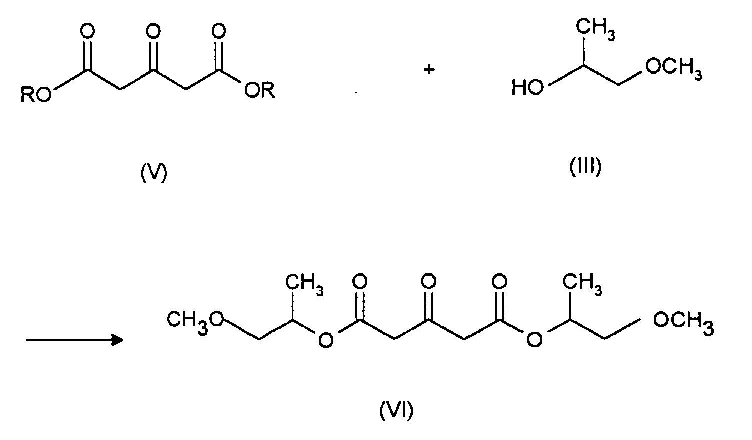 und das Umesterungsprodukt dann mit 4-Diethylaminosalicylaldehyd der Formel VII umgesetzt wird.

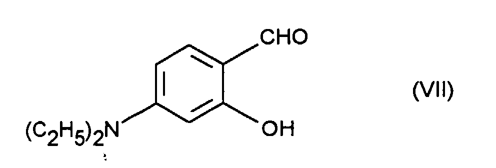

Beispiele für den Alkylrest R sind Methyl, Ethyl, n-Propyl, Isopropyl, n-Butyl, Isobutyl, sec-Butyl und tert.-Butyl. Bevorzugt steht R für Methyl oder Ethyl, besonders bevorzugt für Methyl. In der besonders bevorzugten Ausführungsform dieser Erfindung wird damit Acetondicarbonsäuredimethylester eingesetzt und es werden 3-(7-Diethylamino-2-oxo-2H-chromen-3-yl)-3-oxopropionsäure-(1-methoxy-2-propylester) der Formel I oder Gemische aus 3-(7-Diethylamino-2-oxo-2H-chromen-3-yl)-3-oxopropionsäure-(1-methoxy-2-propylester) der Formel I und 3-(7-Diethylamino-2-oxo-2H-chromen-3-yl)-3-oxopropionsäuremethylester der Formel III hergestellt.

Die in der ersten Stufe des erfindungsgemäßen Verfahrens erfolgende Umesterung des Acetondicarbonsäuredialkylesters der allgemeinen Formel V kann analog bekannten Umesterungsmethoden erfolgen. Bevorzugt erfolgt sie gemäß J. Am. Chem. Soc. 73 (1951), 4195 unter neutralen Bedingungen unter Abdestillieren des Alkohols ROH. Dazu wird ein Gemisch aus der Verbindung der allgemeinen Formel V und 1-Methoxy-2-propanol der Formel III erhitzt, beispielsweise auf eine Temperatur von 90 bis 140°C. Der Umsetzungsgrad kann anhand der abdestillierten Menge des Alkohols ROH oder gaschromatographisch verfolgt werden und im Hinblick auf optimale anwendungstechnische Eigenschaften des herzustellenden PhotoresistFarbstoffes in weiten Grenzen variiert werden. Der Ester der Formel VI bzw. das Gemisch aus dem Ester der Formel VI, dem Ester der allgemeinen Formel V und dem entsprechenden gemischten Ester kann zwischenisoliert werden, z.B. durch Destillation, es kann aber auch das Umesterungsprodukt oder auch das gesamte Reaktionsgemisch der Umesterungsreaktion direkt in die zweite Stufe des Verfahrens eingesetzt werden. Gewünschtenfalls kann nach üblichen Methoden, beispielsweise durch Destillation oder Chromatographie, aus dem Reaktionsgemisch der in der ersten Stufe durchgeführten Umesterung der reine Acetondicarbonsäuredi-(1-methoxy-2-propylester) der Formel VI isoliert werden. Gegenstand der vorliegenden Erfindung ist auch der Acetondicarbonsäuredi-(1 - methoxy-2-propylester) der Formel VI sowie seine Verwendung zur Herstellung des 1-Methoxy-2-propylesters der Formel I.

Die in der zweiten Stufe des erfindungsgemäßen Verfahrens erfolgende Umsetzung des Umesterungsproduktes mit 4-Diethylaminosalicylaldehyd der Formel VII kann gemäß der Vorgehensweise in der DE-C-2 704 368 in einem inerten organischen Lösungsmittel unter Basenkatalyse erfolgen. Bevorzugte Lösungsmittel sind Ether, Alkohole und teilweise veretherte mehrwertige Alkohole. Als Beispiele seien genannt Dialkylether wie Dipropyl- oder Dibutylether, cyclische Ether wie Tetrahydrofuran oder Dioxan, Mono- und Dialkylether des Ethylenglykols und Propylenglykols und von Di- und Oligoethylen- und -propylenglykolen wie Ethylenglykolmono- oder -dimethylether oder Propylenglykolmonomethylether, oder Alkanole wie Methanol, Ethanol, n-Propanol, Isopropanol oder n-Butanol. Ein besonders bevorzugtes Lösungsmittel ist 1-Methoxy-2-propanol. Es können auch Lösungsmittelgemische, insbesondere Gemische der genannten Lösungsmittel, und auch Gemische aus einem oder mehreren organischen Lösungsmitteln mit Wasser eingesetzt werden.

Als Basen eignen sich organische Basen wie z.B. Amine und anorganische Basen wie z.B. Alkali- und Erdalkalimetallhydroxide, Erdalkalimetalloxide und die Carbonate und Hydrogencarbonate von Alkali- und Erdalkalimetallen, wobei als Beispiele für diese Metalle Lithium, Natrium, Kalium, Cäsium, Magnesium, Calcium und Barium genannt seien. Bevorzugt werden organische Basen eingesetzt, weil dadurch die Gegenwart von Metallspuren im Produkt ausgeschlossen wird, die bei der Verwendung des Produkts in Photoresists stören können. Besonders bevorzugt sind als Basen sekundäre Amine, beispielsweise Diethylamin, Pyrrolidin, Piperidin und Morpholin, ganz besonders bevorzugt sind Morpholin und Piperidin. Es können auch Gemische von zwei oder mehreren Basen eingesetzt werden. Die Menge der Base hängt vom Einzelfall ab und kann je nach dem gewünschten Ablauf der Reaktion in weiten Grenzen variiert werden. Im allgemeinen werden 0.05 bis 2.5 Mol Base, bevorzugt 0.1 bis 1 Mol Base, besonders bevorzugt 0.1 bis 0.6 Mol Base pro Mol 4-Diethylaminosalicylaldehyd der Formel VII eingesetzt.

Die Umsetzung des Umesterungsproduktes der ersten Stufe mit dem 4-Diethylaminosalicylaldehyd der Formel VII erfolgt in der üblichen Weise. Das Umesterungsprodukt kann dabei in Form des Reaktionsgemisches der ersten Stufe, also ohne jede Aufarbeitung nach dem Umesterungsschritt, in Form eines zwischenisolierten oder nicht zwischenisolierten Rohproduktes oder in gereinigter Form eingesetzt werden. Beispielsweise kann das Umesterungsprodukt mit dem Aldehyd der Formel VII und dem Lösungsmittel gemischt werden, das Gemisch dann unter Rühren auf die gewünschte Temperatur gebracht werden und bei dieser Temperatur die Base in einer Portion oder langsam während eines längeren Zeitraums zugesetzt werden. Das Reaktionsgemisch wird dann bei der Reaktionstemperatur gehalten, bis die Umsetzung beendet oder der gewünschte Umsetzungsgrad erreicht ist. Die katalytisch wirksame Base kann aber ebenso auch vor dem Einstellen der Reaktionstemperatur oder während eines Aufheizvorganges zugesetzt werden. Ebenso kann beispielsweise nur das Umesterungsprodukt oder nur der Aldehyd der Formel VII mit dem Lösungsmittel vorgelegt werden und der andere Reaktionspartner und auch die Base anschließend bei der gewünschten Temperatur zudosiert werden. Die Reaktion wird im allgemeinen bei Atmosphärendruck zwischen Raumtemperatur und der Rückflußtemperatur des Lösungsmittels bzw. des Reaktionsgemisches durchgeführt. Sie kann aber auch bei höheren Temperaturen und unter Druck durchgeführt werden, und es kann auch die Temperatur während der Umsetzung geändert werden, beispielsweise zur Vervollständigung der Reaktion gegen Ende nochmals erhöht werden. Bevorzugt wird die zweite Stufe des erfindungsgemäßen Verfahrens bei 40 bis 90°C, besonders bevorzugt bei 50 bis 80°C durchgeführt. Die Umsetzung ist im allgemeinen nach 0.5 bis 6 Stunden beendet.

Zweckmäßigerweise wird in der zweiten Stufe der Acetondicarbonsäureester der Formel VI bzw. das nach der Umesterung vorliegende Gemisch der Ester in geringem Überschuß, bezogen auf den Aldehyd der Formel VII, eingesetzt. Bevorzugt werden 0.8 bis 0.95 Mol 4-Diethylaminosalicylaldehyd der Formel VII pro Mol Ester bzw. Estergemisch eingesetzt.

Die Aufarbeitung des Reaktionsgemisches der zweiten Stufe kann analog den oben im Abschnitt zur Umesterung von II zu I beschriebenen Verfahren erfolgen, z.B. durch Abkühlen und/oder Ausfällen, gewünschtenfalls jeweils nach teilweisem Einengen, und Zentrifugieren oder Abfiltrieren des auskristallisierten 1-Methoxy-2-propylesters der Formel I bzw. des Gemisches aus dem Ester der Formel I und dem Ester der allgemeinen Formel IV. Gewünschtenfalls kann auch hier wieder aus einem Gemisch der reine Ester der Formel I nach den üblichen Methoden isoliert werden. In bevorzugter und besonderes einfacher Weise kann auch hier die Aufarbeitung durch Abkühlen in Gegenwart eines zusätzlichen organischen Lösungsmittels als Fällungsmittel erfolgen, insbesondere in Gegenwart von Methanol, das zu einer verbesserten, besonders gut filtrierbaren und waschbaren Kristallform führt. Ganz besonders bevorzugt wird das Reaktionsgemisch in der Wärme mit Methanol versetzt und dann auf 0 bis 20°C, insbesondere auf 5 bis 10°C, abgekühlt.

Nach dem erfindungsgemäßen Verfahren werden in guten Ausbeuten von ca. 70 % der 1-Methoxy-2-propylester der Formel I bzw. Gemische aus dem 1-Methoxy-2-propylester der Formel I und dem Alkylester der allgemeinen Formel IV, die hohe Gehalte an 1-Methoxy-2-propylester von über 90 % aufweisen, auf technisch einfache Weise erhalten. Die Produkte zeichnen sich insbesondere durch eine hohe Reinheit aus. Die Raumausbeute liegt bei unter 10 l/kg.

### Beispiele

### Vergleichsbeispiel (gemäß Beispiel 4 in der US-A-5 225 312)

150 g (0.473 Mol) 3-(7-Diethylamino-2-oxo-2H-chromen-3-yl)-3-oxopropionsäuremethylester werden in 1125 g (12.5 Mol) 1-Methoxy-2-propanol vorgelegt und mit 60 g (0.6 Mol) 98proz. Schwefelsäure versetzt. Man heizt auf 80°C und hält 3 Stunden bei dieser Temperatur. Dann werden unter Anlegen von Vakuum flüchtige Bestandteile bei 80°C abdestilliert. Nach insgesamt 6 Stunden bei 80°C wird auf 50°C gekühlt und mit 250 g (2.87 Mol) Morpholin zur Neutralisation der Schwefelsäure versetzt. Die Farbstofflösung wird dann unter starkem Rühren zu 2 l Wasser von 10°C gegeben. Das auskristallisierte Produkt wird abgesaugt und mit 1 l Wasser portionsweise gewaschen. Das Produkt wird im Vakuum getrocknet.
Ausbeute: 130 g (86.7 Gewichtsprozent, bezogen auf das Gewicht des eingesetzten Methylesters)
Nach dem Hochdruckflüssigkeitschromatogramm enthält das Produkt 16.6 Flächenprozent 3-(7-Diethylamino-2-oxo-2H-chromen-3-yl)-3-oxopropionsäure-(1-methoxy-2-propylester) und 44.5 Flächenprozent 3-(7-Diethylamino-2-oxo-2H-chromen-3-yl)-3-oxopropionsäuremethylester sowie zahlreiche Verunreinigungen.

### Beispiel 1

150 g (0.473 Mol) 3-(7-Diethylamino-2-oxo-2H-chromen-3-yl)-3-oxopropionsäuremethylester werden in 280 g (3.11 Mol) 1-Methoxy-2-propanol vorgelegt und auf 110°C erhitzt. Nach 2 Stunden bei 110°C wird auf 70°C abgekühlt und das Reaktionsgemisch im Laufe von 2 Stunden bei 60 - 70°C mit 280 g Methanol versetzt. Dann kühlt man auf 5°C, saugt das auskristallisierte Produkt ab und wäscht mit Methanol. Das Produkt wird im Vakuum getrocknet. Ausbeute: 150.8 g (100.5 Gewichtsprozent, bezogen auf das Gewicht des eingesetzten Methylesters)
Nach dem Hochdruckflüssigkeitschromatogramm enthält das Produkt 55.9 Flächenprozent 3-(7-Diethylamino-2-oxo-2H-chromen-3-yl)-3-oxopropionsäure-(1-methoxy-2-propylester) und 44.1 Flächenprozent 3-(7-Diethylamino-2-oxo-2H-chromen-3-yl)-3-oxopropionsäuremethylester.

### Beispiel 2

Ein Gemisch aus 34.8 g (0.2 Mol) Acetondicarbonsäuredimethylester und 54 g (0.6 Mol) 1-Methoxy-2-propanol wird auf eine Innentemperatur von 120 - 140°C erhitzt. Bei dieser Temperatur werden über eine Kolonne (15 cm; Raschig-Ringe) 12 ml (ca. 0.3 Mol) Methanol abdestilliert. Zu der resultierenden Mischung gibt man 300 ml 1-Methoxy-2-propanol und trägt 36.5 g (0.19 Mol) 4-Diethylaminosalicylaldehyd ein. Nach 15minütigem Rühren bei Raumtemperatur fügt man 9.8 g (0.11 Mol) Morpholin zu, erhitzt innerhalb von 1 Stunde auf 67 - 70°C und rührt 1 Stunde bei dieser Temperatur nach. Dann kühlt man auf 5 - 10°C, saugt das ausgefallene Produkt ab, wäscht es mit Methanol und trocknet.
Ausbeute: 47.9 g
Nach dem Hochdruckflüssigkeitschromatogramm enthält das Produkt 92.7 Flächenprozent 3-(7-Diethylamino-2-oxo-2H-chromen-3-yl)-3-oxopropionsäure-(1-methoxy-2-propylester) und 6.6 Flächenprozent 3-(7-Diethylamino-2-oxo-2H-chromen-3-yl)-3-oxopropionsäuremethylester.

### Beispiel 3

Ein Gemisch aus 34.8 g (0.2 Mol) Acetondicarbonsäuredimethylester und 54 g (0.6 Mol) 1-Methoxy-2-propanol wird auf eine Innentemperatur von 120 - 140°C erhitzt. Bei dieser Temperatur werden über eine Kolonne (15 cm; Raschig-Ringe) 6 ml (ca. 0.15 Mol) Methanol abdestilliert. Zu der resultierenden Mischung gibt man 300 ml 1-Methoxy-2-propanol und trägt 36.5 g (0.19 Mol) 4-Diethylaminosalicylaldehyd ein. Nach 15minütigem Rühren bei Raumtemperatur fügt man 9.8 g (0.11 Mol) Morpholin zu, erhitzt innerhalb von 1 Stunde auf 67 - 70°C und rührt 1 Stunde bei dieser Temperatur nach. Dann verdünnt man mit 300 ml Methanol, kühlt auf 5 - 10°C, saugt das ausgefallene Produkt ab, wäscht es mit Methanol und trocknet.
Ausbeute: 46.9 g
Nach dem Hochdruckflüssigkeitschromatogramm enthält das Produkt 63.5 Flächenprozent 3-(7-Diethylamino-2-oxo-2H-chromen-3-yl)-3-oxopropionsäure-(1-methoxy-2-propylester) und 35.5 Flächenprozent 3-(7-Diethylamino-2-oxo-2H-chromen-3-yl)-3-oxopropionsäuremethylester.

### Beispiel 4

Ein Gemisch aus 34.8 g (0.2 Mol) Acetondicarbonsäuredimethylester und 54 g (0.6 Mol) 1-Methoxy-2-propanol wird auf eine Innentemperatur von 120 - 140°C erhitzt. Bei dieser Temperatur werden über eine Kolonne (15 cm; Raschig-Ringe) 12 ml (ca. 0.3 Mol) Methanol abdestilliert. Die resultierende Mischung wird im Vakuum zunächst von überschüssigem 1-Methoxy-2-propanol befreit und dann fraktioniert destilliert.
¹H-NMR-Spektrum des als Hauptkomponente erhaltenen Acetondicarbonsäuredi-(1-methoxy-2-propylester) (in D₆-DMSO; δ (ppm)):
   1.2 (d, 6H, CH₃-CH-), 3.3 (s, 6H, CH₃-O-), 3.4 (d, 4H, -CH₂-O-), 3.7 (s, 4H, -CO-CH₂-CO-), 5.0 (m, 2H, O-CH(CH₃)CH₂-).

## Patentansprüche

1. Verfahren zur Herstellung von 3-(7-Diethylamino-2-oxo-2H-chromen-3-yl)-3-oxopropionsäure-(1-methoxy-2-propylester) der Formel I oder von Gemischen aus 3-(7-Diethylamino-2-oxo-2H-chromen-3-yl)-3-oxopropionsäure-(1-methoxy-2-propylester) der Formel I und 3-(7-Diethylamino-2-oxo-2H-chromen-3-yl)-3-oxopropionsäuremethylester der Formel II durch Umesterung von 3-(7-Diethylamino-2-oxo-2H-chromen-3-yl)-3-oxopropionsäuremethylester der Formel II mit 1-Methoxy-2-propanol der Formel III, 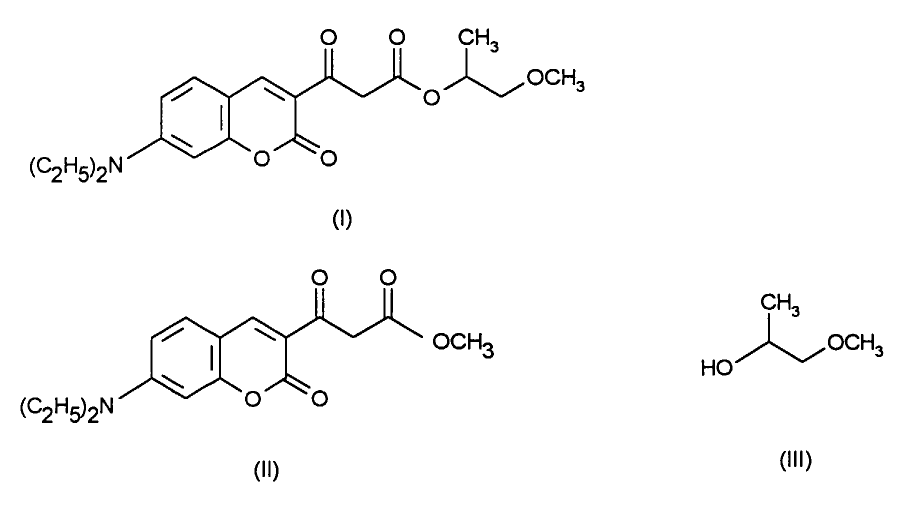 dadurch gekennzeichnet, daß bei der Umesterung kein Katalysator zugesetzt wird.

2. Verfahren gemäß Anspruch 1 , dadurch gekennzeichnet, daß die Umesterung bei 80 bis 150°C, bevorzugt bei 90 bis 130°C, besonders bevorzugt bei 100 bis 120°C, durchgeführt wird.

3. Verfahren gemäß Anspruch 1 und/oder 2, dadurch gekennzeichnet, daß pro Mol Methylester der Formel II 5 bis 10 Mol, bevorzugt 6 bis 8 Mol, 1-Methoxy-2-propanol der Formel III eingesetzt werden.

4. Verfahren gemäß einem oder mehreren der Ansprüche 1 bis 3, dadurch gekennzeichnet, daß zur Isolierung des Umesterungsproduktes dem Reaktionsgemisch Methanol zugesetzt wird.

5. Verfahren zur Herstellung von 3-(7-Diethylamino-2-oxo-2H-chromen-3-yl)-3-oxopropionsäure-(1-methoxy-2-propylester) der Formel I oder von Gemischen aus 3-(7-Diethylamino-2-oxo-2H-chromen-3-yl)-3-oxopropionsäure-(1-methoxy-2-propylester) der Formel I und 3-(7-Diethylamino-2-oxo-2H-chromen-3-yl)-3-oxopropionsäurealkylester der allgemeinen Formel IV, in der R für (C₁-C₄)-Alkyl steht, 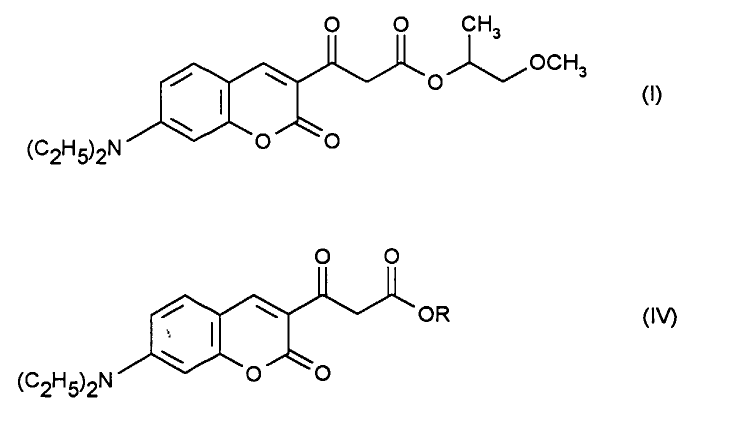 dadurch gekennzeichnet, daß zunächst ein Acetondicarbonsäuredialkylester der allgemeinen Formel V, in der R für (C₁-C₄)-Alkyl steht, mit 1-Methoxy-2-propanol der Formel III zu Acetondicarbonsäuredi-(1-methoxy-2-propylester) der Formel VI oder zu einem Gemisch aus dem Ester der Formel VI, dem Ester der allgemeinen Formel V und dem entsprechenden gemischten Ester umgeestert wird 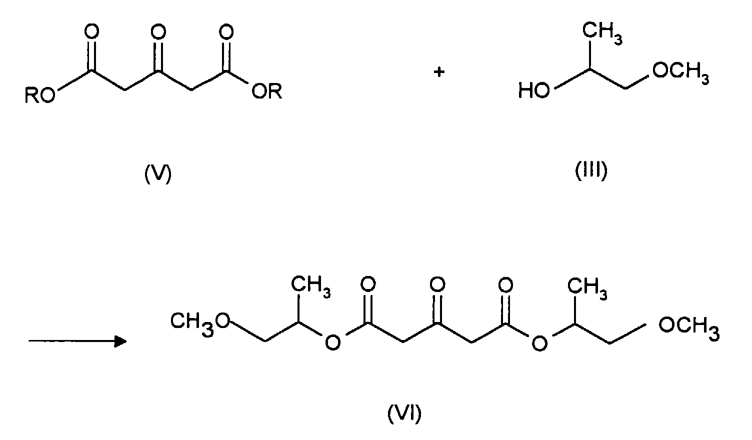 und das Umesterungsprodukt dann mit 4-Diethylaminosalicylaldehyd der Formel VII umgesetzt wird.

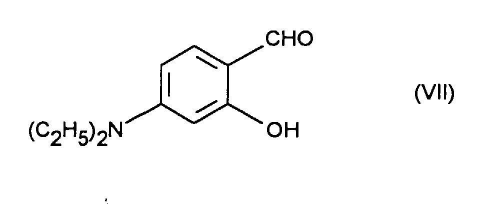

6. Verfahren gemäß Anspruch 5, dadurch gekennzeichnet, daß R für Methyl oder Ethyl, bevorzugt Methyl, steht.

7. Verfahren gemäß Anspruch 5 und/oder 6, dadurch gekennzeichnet, daß das zunächst erhaltene Umesterungsprodukt nicht zwischenisoliert wird.

8. Verfahren gemäß einem oder mehreren der Ansprüche 5 bis 7, dadurch gekennzeichnet, daß die Umsetzung des Umesterungsproduktes mit dem Aldehyd der Formel VII unter Basenkatalyse, bevorzugt in Gegenwart eines sekundären Amins, besonders bevorzugt in Gegenwart von Morpholin oder Piperidin, durchgeführt wird.

9. Verfahren gemäß einem oder mehreren der Ansprüche 5 bis 8, dadurch gekennzeichnet, daß zur Isolierung des Produktes dem Reaktionsgemisch Methanol zugesetzt wird.

10. Acetondicarbonsäuredi-(1-methoxy-2-propylester).
